(19) Europäisches Patentamt
European Patent Office
Office européen des brevets

(11) **EP 2 572 702 B1**

(12) **FASCICULE DE BREVET EUROPEEN**

(45) Date de publication et mention
de la délivrance du brevet:
**08.10.2014 Bulletin 2014/41**

(21) Numéro de dépôt: **12196144.5**

(22) Date de dépôt: **08.12.2008**

(51) Int Cl.:
*A61K 8/73* (2006.01)     *A61K 31/738* (2006.01)
*A61L 27/20* (2006.01)     *A61L 27/26* (2006.01)
*A61L 27/52* (2006.01)

(54) **Hydrogel cohésif monophasique biodégradable**

Bioabbaubares monophasisches kohäsives Hydrogel

Biodegradable single-phase cohesive hydrogel

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **07.12.2007 FR 0759641**

(43) Date de publication de la demande:
**27.03.2013 Bulletin 2013/13**

(62) Numéro(s) de document de la (des) demande(s)
initiale(s) en application de l'article 76 CBE:
**08858227.5 / 2 231 108**

(73) Titulaire: **Laboratoires Vivacy**
**74160 Archamps (FR)**

(72) Inventeurs:
• **Piron, Estelle**
  **73130 Saint Etienne de Cuines (FR)**
• **Vitally, Guy**
  **73370 Le Bourget du Lac (FR)**

(74) Mandataire: **Tripoz, Inès**
**CABINET Inès Tripoz**
**52, rue du Colombier**
**69007 Lyon (FR)**

(56) Documents cités:
**WO-A-2005/061611     FR-A- 2 733 427**
**FR-A- 2 865 737**

## Description

L'invention concerne le domaine des hydrogels biodégradables réticulés ayant des applications en esthétique, par exemple ou en médecine.

Parmi les applications esthétiques on citera par exemple le comblement des ridules, rides et défauts cutanés et l'augmentation des volumes.

Parmi les applications médicales on citera par exemple l'injection périurétrale pour le traitement de l'incontinence urinaire par insuffisance sphinctérienne, l'injection post chirurgicale pour éviter les adhésions péritonéales notamment ; l'injection pour remplacement de liquides biologiques déficients (dans les articulations notamment pour remplacer le liquide synovial déficient), et l'injection suite à une chirurgie de la presbytie par incisions sclérales au laser.

Dans toutes ces applications les hydrogels utilisés doivent présenter des propriétés optimisées en terme de rémanence in vivo, de rhéologie et en terme de viscosité pour garantir une bonne « injectabilité, » ces hydrogels étant appliqués par injection à l'aide d'aiguilles de tailles variables selon les applications, mais qui doivent rester les plus fines possibles afin de minimiser les réactions post-injection.

L'optimisation de ces différentes propriétés conduit à des compromis souvent peu satisfaisants car elles sont parfois incompatibles. En effet pour augmenter la rémanence in-vivo il convient d'augmenter le taux de réticulation, mais en augmentant le taux de réticulation on diminue nécessairement l' « injectabilité ».

De nombreuses solutions ont été proposées et parmi celles-ci on citera les compositions à base de particules permanentes ou très lentement biodégradables dispersées dans un vecteur d'injection, par exemple les particules de PMMA (polyméthacrylate de méthyle) dans un gel de collagène (Artecoll), les particules d'hydrogel acrylique dans un gel de hyaluronate de sodium réticulé (Dermalive, Dermadeep), les particules d'acide polylactique ou polylactide (PLA) dans un vecteur aqueux (New Fill, Sculptra, le PLA étant résorbé en 1 à 4 ans en fonction de la taille des particules).

Ces implants sont sujets à controverse du fait des effets secondaires potentiels dus aux particules solides surtout si elles ne sont pas rondes et si elles ont un caractère permanent. Parmi les complications répertoriées on peut citer les inflammations, les oedèmes et les granulômes.

On peut également citer les implants biodégradables à base de polysaccharides réticulés ou non, essentiellement à base de hyaluronate de sodium.

Pour contourner ces inconvénients, dans la plupart des documents de l'art antérieur par exemple dans la demande FR2865737 déposée par ANTEIS SA ou dans FR2861734 déposée par CORNEAL Industrie SA les produits décrits sont obtenus par une réticulation mise en oeuvre sur un mélange de polymères, pour obtenir des mélanges présentant les propriétés de rémanence in vivo, de rhéologie et de viscosité souhaitées.

Une autre solution est le recours aux réseaux polymères interpénétrés IPN (Interpenetrating Polymer Network) ou réseaux polymères semiinterpénétrés (semi IPN) qui permettent d'optimiser les propriétés et d'obtenir des compositions présentant les propriétés visées comme par exemple dans la demande WO 2005/061611 déposée par INNOMED qui décrit des compositions de réseaux semiinterpénétrés de polysaccharides obtenus par réticulation d'au moins un polysaccharide en présence d'au moins un autre polysaccharide qui n'est pas soumis à une réticulation, ou dans le brevet US 6 224 893 déposé par le MIT (Massachusetts Institute of Technology) qui décrit des compositions formées d'au moins deux polysaccharides qui sont réticulés ensuite par exemple par irradiation, les polymères étant réticulés indépendamment les uns des autres mais en étant interpénétrés pour former des IPN.

Des compositions biphasiques injectables ont également été proposées. La demande de brevet FR2733427 décrit des compositions qui comprennent une phase continue et une phase dispersée constituée de fragments insolubles d'un hydrogel. La phase continue aqueuse sert de véhicule à l'injection des fragments de la phase dispersée.

Cependant ces différentes solutions ne sont pas complètement satisfaisantes.

S'agissant des gels biphasiques les inconvénients en termes de réactions secondaires ont été décrits dans la littérature, et leur niveau d'injectabilité est irrégulier du fait de la taille des particules qui peut être difficile à maîtriser.

De plus, les mécanismes principaux impliqués dans la dégradation de gels de polysaccharides réticulés sont essentiellement surfaciques et aléatoires, ceci d'autant plus sur les produits biphasiques présentant une surface de dégradation plus importante.

S'agissant des produits obtenus par mise en oeuvre d'une réticulation sur un mélange comme ceux décrits dans la demande FR 2 865 737 ou FR 2 861 734 ou des IPN ou semi IPN, bien que leur parfaite monophase ou l'interpénétration des réseaux garantissent une bonne rémanence in vivo et peu ou pas de réaction secondaire, ils ne permettent pas d'apporter une solution satisfaisante car la mise en oeuvre des réactions de réticulation parfois sélectives sur des mélanges notamment de polymères naturels présente des difficultés techniques en raison par exemple des variations de masse moléculaire. Ces produits ne permettent pas de garantir une reproductibilité parfaite des propriétés physiques d'un lot à l'autre d'où une industrialisation difficile.

La présente invention permet de résoudre ces différents inconvénients.

La présente invention consiste en un hydrogel cohésif monophasique biodégradable selon la revendication 1.

La cohésivité et le caractère monophasique d'un gel selon l'invention s'entend de la propriété dudit gel de conserver sa stabilité et son unité sans possibilité de séparer les gels constitutifs.

On entend par mélange une juxtaposition de x polymères sans création de liaison covalente entre ceux-ci. Du fait de la présence de groupements polaires et du milieu aqueux, des interactions se créent entre les différents polymères, ces interactions sont du type liaisons faibles de basse énergie faisant intervenir des forces comme par exemple des ponts hydrogène intermoléculaires voire des liaisons ioniques.

Le mélange ainsi obtenu présente des propriétés comparables à celles des IPN sans être un IPN au sens de la définition de l'IUPAC en effet cette définition exclut les mélanges de réseaux préalablement réticulés, Ici, cependant les gels préalablement réticulés et cohésifs se mêlent intimement, en générant des liaisons inter-chaînes faibles entre eux.

Ils deviennent indissociables l'un de l'autre, générant ainsi un réseau de gels réticulés entrelacés dont la cohésivité est voisine de celle des IPN.

Un tel produit présente les avantages des réseaux IPN sans les inconvénients de mise en oeuvre de ceux-ci et permet grâce à l'utilisation d'un taux de réticulation différent pour chaque gel constitutif (ou identique si les gels ont des masses moléculaires très différentes) de créer des réseaux plus ou moins denses avant leur hydratation finale et après mélange d'obtenir un produit dont les propriétés rhéologiques pourront être « réglées » par mesure des propriétés des différents gels constitutifs avant le mélange.

Les réactions de réticulation peuvent ainsi être mises en oeuvre sur des polymères isolés donc en évitant les problèmes de sélectivité.

La mise en oeuvre est ainsi aisée et permet de répondre aux exigences finales tout en utilisant des produits naturels dont les propriétés, notamment la masse moléculaire, peuvent varier d'un lot à l'autre.

De plus la mise en oeuvre des conditions de réticulation est simple, chaque gel étant réticulé indépendamment l'un de l'autre.

Le mélange ainsi obtenu alliera les avantages de chacun des différents gels constitutifs en minimisant leurs inconvénients sans provoquer les effets secondaires observés avec l'utilisation des compositions à base de particules.

On observera une optimisation et une synergie dans les propriétés résultantes tant en terme d'injectibilité qu'en terme de rémanence.

La synergie est obtenue du fait de l'optimisation des deux paramètres qui agissent réciproquement l'un sur l'autre, la faible réticulation favorisant l'injectabilité et défavorisant la rémanence et la forte réticulation favorisant la rémanence et a contrario défavorisant l'injectabilité.

Les propriétés respectives des réseaux se complètent, ainsi le gel de paramètre élastique le plus élevé va induire une augmentation du paramètre élastique de l'ensemble en comparaison au gel le moins réticulé. En revanche, pour l'injectabilité, (reliée à la viscosité du produit), le gel de niveau d'injectabilité le plus faible va permettre de diminuer le niveau d'injectabilité de l'ensemble. On optimise ainsi les caractéristiques du mélange final, avec une synergie des paramètres élastique et visqueux des mélanges obtenus, adaptables en fonction des proportions respectives de chacun des gels constitutifs, et de la pathologie visée

Il est ainsi possible d'adapter la viscosité du mélange en ajustant la proportion de chacun des x polymères. Dans le cas d'un mélange final trop fluide l'ajout d'un polymère fortement réticulé permettra l'obtention d'un niveau d'injectabilité convenable. A l'inverse, dans le cas d'un mélange final trop visqueux l'ajout d'un polymère faiblement réticulé permettra de diminuer le taux d'injectabilité de l'ensemble.

Ainsi, quelle que soit l'application visée, l'utilisation d'aiguilles plus fines que celles généralement utilisées permettra de diminuer les réactions inflammatoires et les traumatismes post-injection.

Les caractéristiques étant reproductibles, la rémanence du gel sera connue et prédictible aux facteurs de variation inter-individu près et la reproductibilité des propriétés d'injectabilité permettra une grande maîtrise du geste et l'élimination d'un certain nombre d'effets secondaires.

Un hydrogel, de par sa constitution, présentera une cinétique de dégradation fonction du nombre de gels mélangés et des taux de réticulation. En effet, la cinétique de dégradation est fonction de plusieurs paramètres : le taux de réticulation, la concentration en polymère ainsi que la masse moléculaire des polymères utilisés au moment de la réticulation.

La cinétique de dégradation sera ralentie ; en effet mélanger de manière homogène des gels de degrés de rémanence variables permettra de renforcer la rémanence globale par un effet de « dilution » des coupures aléatoires du gel par l'intermédiaire soit des radicaux libres, soit des enzymes (hyaluronidases, etc...) présentes dans le derme ou le liquide biologique remplacé. Le produit fini ainsi fabriqué sera donc plus rémanent pour des niveaux d'injectabilité équivalents, ceci tout en restant parfaitement biodégradable.

La rémanence sera également optimisée du fait de l'interpénétration des réseaux, augmentant la densité de noeuds ou liaisons chimiques tout en conservant l'indépendance mécanique et chimique des 2 à x gels réticulés. Ainsi l'attaque aléatoire des radicaux libres est statistiquement plus faible par rapport à un gel monophasique simple (1 seul réseau, fragilisation des liaisons plus rapides en surface, densité de liaisons chimiques plus faible). L'accessibilité au coeur du gel sera également rendue beaucoup plus difficile pour les dégradations enzymatiques ou via les CD44. Par ailleurs, l'utilisation de différentes masses moléculaires dans chaque gel monophasique préalablement réticulé permettra de constituer des réseaux de structure ou maillage plus ou moins dense et ainsi de renforcer encore la rémanence in vivo.

L'hydrogel selon l'invention est caractérisé en ce que les polymères sont choisis parmi les polysaccharides.

Les polysaccharides sont choisis dans le groupe constitué par l'acide hyaluronique, le kératane, l'héparine, la cellulose et les dérivés de cellulose, l'acide alginique, le xanthane, la carraghénane, le chitosane, la chondroitine et leurs sels biologiquement acceptables.

Dans un mode de réalisation au moins un des x polysaccharides est choisi dans le groupe constitué par l'acide hyaluronique et ses sels biologiquement acceptables.

Dans un autre mode de réalisation l'hydrogel selon l'invention est caractérisé en ce que au moins un des x polysaccharides est choisi dans le groupe constitué par les dérivés de cellulose et leurs sels biologiquement acceptables.

Dans un autre mode de réalisation l'hydrogel selon l'invention est caractérisé en ce que au moins un des x polysaccharides est choisi dans le groupe constitué par la chondroitine et ses sels biologiquement acceptables.

Dans un autre mode de réalisation l'hydrogel selon l'invention est caractérisé en ce que au moins un des x polysaccharides est choisi dans le groupe constitué par le chitosane et ses sels et dérivés biologiquement acceptables.

Les polysaccharides qui peuvent être mis en oeuvre dans l'hydrogel selon la présente invention sont de tout type connu dans le domaine et sont de préférence choisis parmi ceux produits par fermentation bactérienne. Généralement les polysaccharides utilisables dans le cadre de la présente invention présentent une masse moléculaire MW comprise entre environ 0,02 et environ 6 MDa, de préférence comprise entre environ 0,04 et environ 4 MDa, de préférence encore comprise entre environ 0,05 et environ 3 MDa.

On préfère particulièrement l'acide hyaluronique et ses sels, en particulier ses sels acceptables du point de vue physiologique, tels que les sels de sodium, potassium, calcium, avantageusement le sel de sodium.

Sont également avantageusement utilisés la chondroïtine sulfate et ses sels et les dérivés cellulosiques tels que l'hydroxypropylméthylcellulose ou la carboxymethylcellulose et les mélanges de deux ou plusieurs d'entre eux.

Le hyaluronate de sodium présentant des propriétés particulièrement avantageuses en raison de son recul important d'utilisation en injection intradermique, intra-articulaire, intrapéritonéale, et autres, ainsi que pour ses excellentes propriétés rhéologiques, les gels constitutifs de l'hydrogel selon l'invention sont de préférence à base de hyaluronate de sodium.

Dans un mode de réalisation les x polymères sont identiques.

Dans un mode de réalisation les x polymères sont différents.

Dans un mode de réalisation, l'hydrogel selon l'invention est caractérisé en ce que x est égal à 2.

Selon un mode de réalisation particulier le premier des x polysaccharides est choisi dans le groupe constitué par l'acide hyaluronique et ses sels, les dérivés de cellulose et leurs sels et la xanthane et le deuxième est choisi dans le groupe constitué par la chondroïtine sulfate et ses sels, le chitosane et ses sels et dérivés, les dérivés de cellulose et leurs sels et les acides alginiques. Dans un autre mode de réalisation particulier, le premier des x polymères est choisi dans le groupe constitué par l'acide hyaluronique et ses sels, les dérivés de cellulose et leurs sels et la xanthane et le deuxième est choisi dans le groupe constitué par les acides polylactiques et leurs dérivés et les dérivés acryliques.

Selon un mode de réalisation, le premier des x polymères est choisi dans le groupe du hyaluronate de sodium et le deuxième est choisi dans le groupe constitué par la chondroïtine sulfate et ses sels, le chitosane et ses sels et dérivés, les dérivés de cellulose et leurs sels et les acides alginiques.

Dans l'hydrogel selon la présente invention, le rapport pondéral entre le polysaccharide fortement réticulé et le polysaccharide faiblement réticulé peut varier dans de très larges proportions, selon la nature des polysaccharides utilisés, leurs taux de réticulation respectifs, et également selon les propriétés finales visées.

Généralement la proportion pondérale du gel de polysaccharide fortement réticulé dans le produit fini est comprise entre environ 0,1 et 99,9 %, préférentiellement de 5 à 50% de gel 1 présentant un taux de réticulation x1 et de 50 à 95 % de gel 2 présentant un taux de réticulation x2 ou encore plus préférentiellement de 10 à 40% de gel 1 présentant un taux de réticulation x1 et de 60 à 90 % de gel 2 présentant un taux de réticulation x2 .

L'invention concerne également le procédé de préparation d'un hydrogel biodégradable selon l'invention ; ce procédé comprend une étape de mise au point d'un cahier des charges fixant les propriétés rhéologiques visées en fonction des applications.

Pour la détermination du taux de rémanence une élasticité est visée, celle-ci résultant du taux de réticulation et pour la détermination de l'injectabilité, la viscosité à taux de cisaillement élevé liée également au taux de réticulation est fixée, ces paramètres dépendent des produits de départ, notamment de leur masse moléculaire.

Les taux de réticulation fixés ainsi que les proportions respectives des gels constitutifs, le procédé de préparation d'un hydrogel cohésif monophasique biodégradable selon l'invention est caractérisé en ce qu'il comprend au moins les étapes de :

- réticulation d'un premier polymère à un taux de réticulation x1
- réticulation d'un deuxième polymère à un taux de réticulation x2
- interpénétration par mélange intime des deux polymères,

- hydratation
- interpénétration finale par mélange final après hydratation.

L'hydratation est effectuée par exemple par immersion dans ou ajout d'une solution isotonique tamponnée.

Selon un mode de réalisation le procédé comprend en outre x étapes de réticulation des x polymères avant le mélange des x polymères réticulés.

L'hydratation est généralement effectuée dans un milieu aqueux, par simple mélange du mélange de gels réticulés avec une solution aqueuse, avantageusement physiologique tamponnée, de manière à obtenir une concentration finale, variable dans de très larges proportions, selon la nature des polysaccharides utilisés, leurs taux de réticulation respectifs, et également selon l'utilisation envisagée. La solution tamponnée utilisable peut par exemple être une solution physiologique, osmolaire présentant un pH compris entre environ 6,8 et environ 7,5.

Cette concentration finale en polysaccharides totaux est généralement comprise entre environ 5 et environ 100 mg/g, de préférence entre environ 5 et environ 50 mg/g par exemple environ 20 mg/g d'hydrogel.

Le procédé de la présente invention permet ainsi d'obtenir un hydrogel cohésif monophasique biodégradable, injectable et de rémanence longue.

Dans le procédé de préparation ci-dessus décrit, les deux étapes de réticulation sont réalisées dans un milieu dont la valeur de pH est identique ou différente. Chacune de ces étapes peut être effectuée en milieu acide ou basique, de préférence en milieu basique, par exemple à un pH compris entre 8 et 14, de préférence entre 8 et 13.

Les réactions de réticulation mises en oeuvre dans le procédé de l'invention sont des réactions bien connues de l'homme de l'art. Pour chaque polysaccharide et/ou agent réticulant, l'homme de l'art pourra mettre au point et optimiser les conditions de réticulations en fonction dudit polysaccharide et dudit agent réticulant : taux de réticulation, température, pH. Il est cependant précisé que les étapes de réticulation sont réalisées à pH constant, soit acide, soit basique, comme indiqué précédemment.

Les agents réticulants qui interviennent dans les étapes de réticulation sont généralement des réticulants bi- ou poly-fonctionnels de différents types, et peuvent par exemple être choisis parmi la DVS (divinylsulfone) en milieu alcalin (voir US 4 582 865), les époxy bi- ou poly-fonctionnels (voir US 4 716 154), les carbodi-imides, le formaldéhyde (voir GB 2 151 244).

On préfère en particulier les agents de type bi- ou poly-époxydes, les réactions se faisant en milieu basique pour générer des liaisons éther avec les fonctions -OH du polysaccharide, ou en milieu acide ce qui donne lieu à des liaisons de type ester. La demande de brevet WO 2000/46253 utilise successivement ces deux conditions de pH afin d'optimiser la réticulation du polysaccharide. On préfère toutefois réaliser les réactions de réticulation en conditions de pH basique, puisque, en milieu aqueux, les liaisons ester, obtenues en milieu acide, sont généralement plus labiles que les liaisons éther, obtenues en milieu basique.

À titre d'agent réticulant, on peut utiliser un époxyde ou ses dérivés, et notamment le 1,4-butanedioldiglycidyléther (BDDE), le diépoxy-octane ou le 1,2-bis-(2,3-époxypropyl)-2,3-éthylène. On préfère tout particulièrement utiliser le 1,4-butanedioldiglycidyléther (BDDE) pour chacune des étapes de réticulation.

Il doit être compris que chacune des étapes de réticulation peut être effectuée avec un ou plusieurs agents réticulants, ceux-ci pouvant être identiques ou différents dans l'une ou l'autre des étapes, dans les conditions de pH indiquées plus haut.

Après chacune des étapes de réticulation, les polysaccharides peuvent avantageusement être purifiés, selon des techniques classiques de purification (par exemple par lavage par flux d'eau continu, bains de dialyse, et autres), afin d'éliminer l'agent de réticulation résiduel n'ayant pas réagi.

En outre, les étapes de réticulation peuvent avantageusement être suivies d'une étape de neutralisation (i.e. jusqu'à une valeur de pH d'environ 7), par exemple par ajout d'une quantité appropriée d'acide chlorhydrique 1 N.

Dans l'hydrogel selon l'invention, les x polymères présentent des taux de réticulation différents, au moins l'un des x polymères présentant un taux de réticulation x1 et au moins un des x polymères présentant un taux de réticulation x2 et x1 étant supérieur à x2.

Dans un mode de réalisation dans l'hydrogel selon l'invention, les x polymères présentent des taux de réticulation identiques. Etant entendu que dans ce cas les polymères ont des masses moléculaires différentes.

Dans un mode de réalisation x1 et x2 sont compris entre 0,02 et 0,4 et de préférence entre 0,08 et 0,2.

À l'issue de la réticulation, il peut être avantageux de neutraliser le gel obtenu, selon les procédés standards connus dans le domaine, et par exemple par ajout d'acide lorsque la réticulation est conduite en milieu basique, et par ajout d'une base, lorsque la réticulation est conduite en milieu acide.

Le mélange obtenu à l'issue du procédé peut être éventuellement soumis à une étape d'hydratation complémentaire, afin d'obtenir un gel sous forme d'hydrogel injectable, adapté aux applications envisagées.

L'invention concerne l'utilisation d'un hydrogel selon l'invention, pour la formulation d'une composition de viscosupplémentation.

L'invention concerne l'utilisation d'un hydrogel selon l'invention, pour la formulation d'une composition pour le comblement des rides.

Les applications visées sont plus particulièrement les applications communément observées dans le cadre des viscoélastiques injectables de polysaccharides utilisés ou potentiellement utilisables dans les pathologies ou traitements suivants :

- injections esthétiques : de comblement des rides, défauts cutanés ou volumatrices (pommettes, mentons, lèvres) ;
- traitement de l'arthrose, injection dans l'articulation en remplacement ou complément du liquide synovial déficient ;
- injection péri-urétrale pour le traitement de l'incontinence urinaire par insuffisance sphinctérienne ;
- injection post chirurgicale pour éviter les adhésions péritonéales notamment ;
- injection suite à une chirurgie de la presbytie par incisions sclérales au laser ;
- injection dans la cavité vitréenne.

Plus particulièrement, en chirurgie esthétique, en fonction de ses propriétés viscoélastiques et de rémanence, l'hydrogel selon l'invention pourra être utilisé :

- pour le comblement des rides fines, moyennes ou profondes, et être injecté avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.

L'hydrogel selon l'invention trouve également une application importante en chirurgie articulaire et en chirurgie dentaire pour le comblement des poches parodontales par exemple.

Ces exemples d'utilisation ne sont nullement limitants, l'hydrogel selon la présente invention étant plus largement prévu pour :

- combler des volumes ;
- générer des espaces au sein de certains tissus, favorisant ainsi leur fonctionnement optimal ;
- remplacer des liquides physiologiques déficients.

L'hydrogel selon l'invention peut également trouver une application tout à fait intéressante en tant que matrice de relargage d'un (ou plusieurs) principe(s) actif(s) au préalable dispersé(s) en son sein. Par principe actif on entend tout produit actif sur le plan pharmacologique : principe actif médicamenteux, antioxydant (sorbitol, mannitol ...), antiseptique, anti-inflammatoire, anesthésiques locaux (lidocaine...) etc.

De manière pratique, l'hydrogel selon l'invention, de préférence après purification et hydratation en hydrogel, peut être conditionné, par exemple dans des seringues, et stérilisé selon tout moyen connu en soi (par exemple par autoclavage) pour commercialisation et/ou utilisation directe.

Selon un autre aspect, la présente invention concerne un kit comprenant un hydrogel selon l'invention, conditionné en seringue stérile.

Les caractéristiques des gels selon l'invention sont mises en évidence dans les exemples ci-après.

**Exemples**

Taux de réticulation :

Les taux de réticulation x dans les exemples qui suivent sont définis par :

x = nombre de moles de réticulant introduites dans le milieu réactionnel / nombre total de motifs disaccharides introduits dans le milieu réactionnel.

**Exemple 1 :**

Réticulation Gel 1

Étape a) : *Hydratation de fibres de hyaluronate de sodium sous forme de gel non réticulé*

Des fibres de hyaluronate de sodium de qualité injectable (1 g ; masse moléculaire : environ 2.7 MDa) ; sont pesées dans un récipient. Une solution aqueuse d'hydroxyde de sodium à 1% dans l'eau (7,4 g) est ajoutée, le tout est homogénéisé pendant environ 1 heure à la spatule, à température ambiante et 900 mm Hg.

Étape b) : *Réticulation*

Du BDDE (65 mg) est ajouté au gel de hyaluronate de sodium (NaHA) non réticulé obtenu à l'étape précédente, le tout étant homogénéisé à la spatule pendant environ 30 minutes à température ambiante. L'ensemble est ensuite placé au bain-marie à 50°C pendant 2h20 afin d'obte nir un taux de réticulation X1 d'environ 0,14.

Etape c) : *Neutralisation, purification*

Le gel final réticulé est ensuite neutralisé par ajout d'HCl 1N, et placé dans un bain de tampon phosphate pour stabiliser le pH et permettre son hydratation, ou gonflement jusqu'à 30 mg/g de HA. On obtient ainsi un hydrogel de NaHa réticulé par la voie classiquement utilisée : G1 de concentration en HA environ 30 mg/g.
Une partie du gel est conservée à cette concentration, l'autre est diluée par ajout de tampon phosphate pour obtenir 20mg/g de HA au final, ce gel est ensuite homogénéisé avant d'être rempli en seringues qui sont stérilisées par autoclavage : seringues de gel G1 à 20 mg/g stériles.

Réticulation Gel 2

Étape a) : *Hydratation de fibres de hyaluronate de sodium sous forme de gel non réticulé*

Des fibres de hyaluronate de sodium de qualité injectable (1 g ; masse moléculaire : environ 1.5 MDa) ; sont pesées et préalablement séchées dans un récipient. Une solution aqueuse d'hydroxyde de sodium à 1% dans l'eau (6,3 g) est ajoutée, le tout est homogénéisé pendant environ 1 heure à la spatule, à température ambiante et 900 mm Hg.

Étape b) : *Réticulation*

Du BDDE (43 mg) est ajouté au gel de hyaluronate de sodium (NaHA) non réticulé obtenu à l'étape précédente, le tout étant homogénéisé à la spatule, à température ambiante et pression atmosphérique pendant environ 30 minutes. L'ensemble est ensuite placé au bain-marie à 50°C pendant 2h20, afin d'obtenir un taux de réticulation X2 d'environ 0.09.

Etape c): Neutralisation, purification

Le gel final réticulé est ensuite neutralisé par ajout d'HCl 1 N, et placé dans un bain de tampon phosphate pour stabiliser le pH et permettre son hydratation, ou gonflement jusqu'à 30 mg/g de HA. On obtient ainsi un hydrogel de NaHa réticulé par la voie classiquement utilisée : G2 de concentration en HA environ 30 mg/g.

**Exemple 2 :**

Mélange/interpénétration des Gel 1 et gel 2 en proportion 10% G1 - 90% G2

*Mélanges/interpénétration des gels G1 et* G2 à *10%* - 90%

18g de gel G2 à 30 mg/g sont pesés, et 2 g de gel G1 obtenu en fin d'étape c) précédente (G1 à 30 mg/g) y sont ajoutés. 10 g de tampon phosphate sont ajoutés et les 2 gels sont placés sous agitation mécanique lente pendant 1h sous pression hyperbare.
Le mélange ainsi obtenu est un gel homogène à 20mg/g de HA, composé de 2 réseaux interpénétrés, ce gel est alors conditionné en seringues et autoclavé.

**Exemple 3 :**

Mélange/interpénétration des Gels 1 et 2 en proportion 50% - 50%
Les gels obtenus en fin d'étape c) de chaque exemple ci-dessus : gel 1 réticulé à X1 environ 0.14 et G2 réticulé à X2 environ 0.09, tous deux de concentration environ 30 mg/g de HA sont pesés : 10 g de G1 + 10g de G2.
10g de tampon phosphate sont également ajoutés et les 2 gels sont placés sous agitation mécanique lente pendant 1 h sous pression hyperbare.
Le mélange ainsi obtenu est un gel homogène à 20mg/g de HA, composé de 2 réseaux interpénétrés, ce gel est alors conditionné en seringues et autoclavé.

**Exemple 4 :**

Caractérisation des gels des exemples 1et 2:

- gel G1 réticulé à X1,
- mélange 10% G1 et 90% G2 réticulé à X2,
- mélange 50% G1 et 50% G2,

Ces 3 produits finaux étant tous les 3 à une concentration finale de 20mg/g en HA.

Caractérisation de la force d'extrusion ou « injectabilité » :
Cet essai est réalisé sur les gels conditionnés en seringues et stérilisés, avec des aiguilles 27G1/2, sur un banc de traction avec une vitesse de compression de 13 mm/min. Les résultats des forces d'extrusion de chacun des exemples 1, 2 et 3 sont donnés dans le tableau ci-dessous :

| Gel testé | Injectabilité (N) |
|---|---|
| Gel 1 | 37 |
| 10% Gel 1 + 90% Gel 2 | 21 |
| 50% Gel 1 + 50% Gel 2 | 31 |

On observe bien une injectabilité plus faible des réseaux interpénétrés de gels réticulés par rapport au gel G1 seul.

Test de dégradation

Ces différents gels ont également été caractérisés par un test de dégradation in vitro à la température. Ce test permet de simuler la rémanence ultérieure in vivo des gels injectés en intra-dermique. Il a été mis au point sur la base du descriptif du test de rémanence décrit dans le brevet FR2861734. Les gels ont tous été placés en étuve à 93°C pendant 14h, 24 h et 48h, avec caractérisation de l'élasticité après chaque temps. Les courbes de tendance des résultats de dégradation de ces différents gels permettant ensuite d'évaluer le temps de ½ vie de ces différents gels (*durée nécessaire pour avoir G' = G'0 / 2, en heures), avec G'0 = élasticité à t0 du gel caractérisé.* Les temps de ½ vie obtenus sont également donnés dans le tableau ci-dessous.

| Gel testé | Temps de ½ vie (heures) |
|---|---|
| Gel 1 | 19 |
| 10% Gel 1 + 90% Gel 2 | 22,5 |
| 50% Gel 1 + 50% Gel 2 | 20,5 |

On observe une dégradation plus importante pour le gel 1 seul en comparaison aux deux réseaux interpénétrés de gels préalablement réticulés.

Ainsi pour une injectabilité inférieure et donc une meilleure maîtrise du geste chirurgical, les temps de demi-vie des réseaux interpénétrés de gels obtenus selon l'invention sont plus longs, garantissant un temps de rémanence in vivo supérieur.

**Exemple 5 :**

Afin de vérifier la cohésivité et le caractère monophasique des hydrogels selon l'invention, des essais de centrifugation manuelle de 3 fois 5 minutes ont été réalisés sur les mélanges 10/90 et 50/50 à 20 mg/g de NaHA obtenus aux exemples précédents.

En comparaison, un produit de type « biphasique », tel que décrit dans l'art antérieur a été réalisé suivant le mode opératoire du brevet EP0466300 avec 50% de particules de NaHa réticulés, dispersées dans 50% de visqueux de NaHA non réticulé, les 2 phases ayant été préalablement hydratées dans du tampon phosphate, à 20 mg/g de NaHA.

Les produits selon l'invention obtenus aux exemples précédents ne montrent aucune décantation, le produit si on l'éjecte après les centrifugations a toujours un aspect homogène.

En revanche, le produit de type « biphasique » après centrifugation montre des particules décantées en fond de seringue. Si on éjecte le produit de la seringue, le visqueux sort le premier, puis les particules qui n'ont aucune cohésivité entre elles, agglomérées en fond de seringue, et qui rendent l'injectabilité particulièrement difficile.

**Exemple 6 :**

Mélange / interpénétration des gels G1 et G2 de l'exemple 1, pour obtention au final de gels et mélanges de gels à une concentration de 25,5 mg/g selon le procédé décrit dans l'exemple 2 avec ajustement des concentrations en NaHa par ajout de tampon phosphate, dans les proportions suivantes :

Gel IPN-Like 1 : 70% Gel 1 rét X1 + 30% Gel 2 rét X2

Gel IPN-Like 2 : 50% Gel 1 rét X1 + 50% Gel 2 rét X2

Gel IPN-Like 3 : 30% Gel 1 rét X1 + 70% Gel 2 rét X2

Ces gels sont alors conditionnés en seringues et stérilisés par autoclavage.

Caractérisation de la force d'extrusion et de l'élasticité de ces gels IPN-Like et du Gel 1 réticulé à X1 et ramené à une concentration en NaHa de 25,5 mg/g :

La force d'extrusion est caractérisée sur banc de traction Mecmesin sous vitesse de compression 50 mm/min avec des aiguilles 23G1 ¼, les résultats sont donnés dans le tableau ci-dessous.

L'élasticité est caractérisée sur rhéomètre TA Instruments AR 2000 Ex, en oscillation à 25°C, la valeur de l'élasticité étant relevée à une fréquence de 1 Hz, les résultats sont donnés dans le tableau ci-dessous.

|  | Gel 1 à 25,5 mg/g | Gels interpénétrés 1 25,5 mg/g | Gels interpénétrés 2 25,5 mg/g | Gels interpénétrés 3 25,5 mg/g |
|---|---|---|---|---|
| Force d'extrusion (N) Aig 23G 1 ¼ Vitesse 50 mm/min | 63 | 61 | 61 | 57 |
| Elasticité : G' (Pa) à 1Hz | 200 | 225 | 244 | 265 |

On observe sur les 3 gels interpénétrés : des forces d'extrusion assez proches mais toutes inférieures au gel 1, pour des élasticités qui croissent. Ainsi, l'utilisation de cette technique d'interpénétration de gels réticulés permet d'obtenir des produits finis de rhéologie variable : élasticité croissante (donc un meilleur effet volumateur et une rémanence attendue supérieure) pour des niveaux d'injectabilité plus faibles.

**Exemple 7 :**

Synthèse gel 3 : Un gel est synthétisé suivant le protocole / conditions opératoires de l'exemple 1, Gel 1 :

Étape a) : *Hydratation de fibres de hyaluronate de sodium sous forme de gel non réticulé*

Cette étape est identique à l'étape a) de synthèse du gel 1 de l'exemple 1.

Étape b) : *Réticulation du gel*

Cette étape est identique à l'étape b) de synthèse du gel 1 de l'exemple 1, avec 81 mg de BDDE. On obtient un Gel 3 de taux de réticulation X3 d'environ 0,17.

Etape c) : *Neutralisation, purification*

Cette étape est Identique à l'étape c) de synthèse du Gel 1 de l'exemple 1, pour obtenir un gel G3 de concentration en HA environ 30 mg/g.

Une partie du gel est conservée à cette concentration, l'autre est diluée par ajout de tampon phosphate pour obtenir 24 mg/g de HA au final, ce gel est ensuite homogénéisé avant d'être rempli en seringues qui sont stérilisées par autoclavage : seringues de gel G3 à 24 mg/g stériles.

Interpénétration Gel 1/ Gel 3 en proportions 80/20:

16g de gel G1 à 30 mg/g sont pesés, et 4 g de gel G3 à 30 mg/g obtenu en fin d'étape c) précédente y sont ajoutés. 5 g de tampon phosphate sont ajoutés et les 2 gels sont placés sous agitation mécanique lente pendant 1 h.

Le mélange ainsi obtenu est un gel homogène à 24 mg/g de HA, composé de 2 réseaux interpénétrés, ce gel est alors conditionné en seringues et autoclavé.

Caractérisation des gels et gels interpénétrés décrits ci-dessus :

- Le gel 3 de taux de réticulation X3, à 24 mg/g,
- le gel 1 de taux de réticulation X1 et préalablement ramené à 24 mg/g, conditionné en seringues et stérilisé,
- et le mélange de gels interpénétrés 80% Gel 1 + 20% Gel 3, à 24 mg/g,

Ces gels sont caractérisés en force d'extrusion. Les essais sont réalisés_avec des aiguilles 27G1/2, sur un banc de traction Mecmesim avec une vitesse de compression de 13 mm/min. Les résultats des forces d'extrusion de chacun de ces gels sont donnés dans le tableau ci-dessous.

Ces gels sont également caractérisés par le test de dégradation in vitro à la température décrit dans l'exemple 4. Les temps de ½ vie obtenus sont également donnés dans le tableau ci-dessous.

| | Force d'extrusion (N) Aiguille 27G1/2- 13 mm/min | Temps de ½ vie (heures) |
|---|---|---|
| Gel 1 - X1 - 24 mg/g | 27 | 17 |
| Gel 3 - X3 - 24 mg/g | 30 | 21 |
| 80% gel 1/20% Gel 3 - 24 mg/g | 23 | 20,5 |

Ainsi, on observe une rémanence équivalente des gels interpénétrés et du gel 3 réticulé au taux le plus élevé X3, ceci pour un niveau d'injectabilité inférieur de ces gels interpénétrés

**Exemple 8 :**

Synthèse de 3 gels réticulés monophasiques suivant les exemples 1 et 2 :

• Gel 4 :

Étape a) : identique à l'étape a) de synthèse du gel 1 de l'exemple 1 avec 1 g de HA de masse moléculaire environ 2,7 MDa et 6,8 g d'une solution aqueuse d'hydroxyde de sodium à 1% dans l'eau. Les conditions d'homogénéisation sont les mêmes que dans l'exemple 1.

Étape b) : *Réticulation :* identique à l'étape b) de synthèse du gel 1 de l'exemple 1 avec 62 mg de BDDE. L'ensemble est porté au bain marie à 50°C pendant 3 heures, pour obtenir un taux de réticulation X4 d'environ 0,13.

Etape c) : *Neutralisation, purification :* identique à l'étape c) de synthèse du gel 1 de l'exemple 1 pour obtenir un gel 4 à 30 mg/g. Une partie du gel est conservée à cette concentration, l'autre est diluée par ajout de tampon phosphate pour obtenir 24 mg/g de HA au final, ce gel est ensuite homogénéisé avant d'être rempli en seringues qui sont stérilisées par autoclavage : seringues de gel G4 à 24 mg/g stériles.

• Gel 5:

Étape a) : identique à l'étape a) de synthèse du gel 4.
Étape b) : *Réticulation:* identique à l'étape b) de synthèse du gel 4 avec 80 mg de BDDE. L'ensemble est porté au bain marie à 50°C pendant 3 heures, pour obtenir un taux de réticulation X5 d'environ 0,17.

Étape c) : *Neutralisation, purification :* identique à l'étape c) de synthèse du gel 4 pour obtenir un gel 5 à 30 mg/g. Une partie du gel est conservée à cette concentration, l'autre est diluée par ajout de tampon phosphate pour obtenir 24mg/g de HA au final, ce gel est ensuite homogénéisé avant d'être rempli en seringues qui sont stérilisées par autoclavage : seringues de gel G5 à 24 mg/g stériles.

• Gel 6 :

Étape a) : Identique à l'étape a) de synthèse du gel 2 de l'exemple 1 avec 1 g de hyaluronate de sodium de masse moléculaire environ 1,3 MDa et 5,7 g d'une solution aqueuse d'hydroxyde de sodium à 1 % dans l'eau.

Étape b) : *Réticulation* Identique à l'étape c) de l'exemple 1 avec 41 mg de BDDE. L'ensemble est porté au bain marie à 50°C pendant 3 heures, pour obtenir un taux de réticulation X6 d'environ 0,09.

Etape c) : *Neutralisation, purification*

Identique à l'étape c) de synthèse du gel 5 précédent pour obtenir un gel 6 à 30 mg/g. Une partie du gel est conservée à cette concentration, l'autre est diluée par ajout de tampon phosphate pour obtenir 24 mg/g de HA au final, ce gel est ensuite homogénéisé avant d'être rempli en seringues qui sont stérilisées par autoclavage : seringues de gel G6 à 24 mg/g stériles.

Interpénétration des gels 4,5 et 6 (proportions respectives : 25%, 5%, 70%)

5 g de gel G4 à 30 mg/g sont pesés, 1 g de gel G5 à 30 mg/g puis 14 g de gel G6 à 30 mg/g. 5 g de tampon phosphate sont ajoutés et les 3 gels sont placés sous agitation mécanique lente pendant 1h. On obtient ainsi un gel final G7 monophasique à 24 mg/g de hyaluronate de sodium composé de 3 gels réticulés monophasiques interpénétrés.

Caractérisation de l'élasticité et de la force d'extrusion des 3 gels classiques_et du_mélange interpénétré :

Selon les méthodes décrites dans les exemples précédents.

| | Gel 4 24 mg/g | Gel 5 24 mg/g | Gel 6 24 mg/g | Gel G7 (interpénétrés 4-5 et 6) 24 mg/g |
|---|---|---|---|---|
| Force d'extrusion (N) Aig 23G 1 ¼ Vitesse 13 mm/min | 31 | 38 | 18 | 16 |
| Elasticité : G' (Pa) à 1Hz | 245 | 415 | 186 | 224 |

Le gel G7 composé de l'interpénétration des 3 gels réticulés (G4, G5 et G6) a la force d'extrusion la plus faible, ceci pour une valeur d'élasticité supérieure de 20% environ à celle du gel G6 de niveau d'injectabilité proche mais légèrement supérieur.

Son élasticité est inférieure de 10% seulement par rapport à celle du gel 4 dont le niveau d'injectabilité est supérieur de plus de 40%.

On note bien l'intérêt de ces gels interpénétrés.

**Exemple 9 :**

Interpénétration de gels HA réticulé et CMC (carboxymethyl cellulose) réticulé

• Gel réticulé de CMC : gel G8

Étape a) : *Hydratation de la CMC Na sous forme de gel non réticulé*

1 g de carboxyméthyl cellulose de sodium de viscosité intrinsèque (fournie par SIGMA) est pesée dans un récipient. Une solution aqueuse d'hydroxyde de sodium à 1 % dans l'eau (7,3 g) est ajoutée, le tout est homogénéisé pendant environ 90 minutes à la spatule, à température ambiante et 900 mm Hg.

Étape b) : *Réticulation*

Du BDDE (37 mg) est ajouté au gel de CMC non réticulé obtenu à l'étape précédente, le tout étant homogénéisé à la spatule pendant environ 30 minutes à température ambiante. L'ensemble est ensuite placé au bain-marie à 50°C pendant 3 h afin d'obtenir un taux de réticulation X8 d'environ 0,19.

Etape c) : *Neutralisation, purification*

Le gel final réticulé est ensuite neutralisé par ajout d'HCl 1 N, et placé dans un bain de tampon phosphate pour stabiliser le pH et permettre son hydratation, ou gonflement jusqu'à 45 mg/g de HA. On obtient ainsi un hydrogel de CMC Na réticulé par la voie classiquement utilisée : G8 de concentration en CMC environ 45 mg/g.

• Interpénétration gel G1 de HA et gel G8 de CMC

Le gel G1 de HA réticulé à un taux de 0,14, à une concentration de 30 mg/g est ajouté en diverses proportions au gel G8 de CMC Na réticulé, du tampon phosphate est ajouté pour ajuster les concentrations finales à 26 mg/g en HA et 37 mg/g en CMC, les 2 gels sont placés sous agitation mécanique lente avec le Tampon phosphate pendant 1 heure sous pression hyperbare. On obtient ainsi 3 gels interpénétrés tels que décrits ci-dessous :

Gel 9 : 30% G1 + 70% G8

Gel 10 : 50% G1 + 50% G8

Gel 11 : 70% G1 + 30% G8

Ces 3 gels interpénétrés sont ensuite conditionnés en seringues et caractérisés en rhéologie (module élastique G') et en injectabilité sous une vitesse 13 mm/min avec une aiguilles 27G1/2. Les gels G1 et G8 sont également ajustés aux concentrations de 26 mg/g pour G1 et 37 mg/g pour G8 afin de les comparer aux 3 gels interpénétrés.

Les résultats des caractérisations sont regroupés dans le tableau ci-dessous.

| | G1 (HA réticulé, 26 mg/g) | G8 (CMC réticulée, 37 mg/g) | G9 Gel interpénétré 30%G1+70%G8 | G10 Gel interpénétré 50%G1+50%G8 | G11 Gel interpénétré 70%G1+30%G8 |
|---|---|---|---|---|---|
| Module élastique G' à 1Hz (Pa) | 235 | 265 | 240 | 243 | 264 |
| Injectabilité Aig 27G1/2(N) | 33 | 18 | 18 | 12 | 16 |

On observe un module élastique quasi constant des 5 gels interpénétrés ou non, mais avec des niveaux d'injectabilité plus faible pour les gels interpénétrés que pour chaque gel réticulé indépendant, avec un effet de synergie important sur le mélange 50/50 (Gel 10).

**Revendications**

**1.**  Hydrogel cohésif monophasique biodégradable **caractérisé en ce que** :

- il est constitué d'un mélange homogène de x polymères choisis parmi les polysaccharides identiques ou différents, réticulés indépendamment l'un de l'autre sous forme d'hydrogel monophasique, préalablement à leur interpénétration par mélange intime générant des liaisons inter-chaînes faibles entre les polymères, lesdits polymères réticulés étant insolubles dans l'eau et miscibles entre eux, et
- x étant compris entre 2 et 5,
- les x polymères réticulés étant indissociables l'un de l'autre,
- les x polymères possédant des degrés de réticulation différents, ou
- les x polymères possédant des degrés de réticulation identiques et des masses moléculaires différentes.

**2.** Hydrogel selon la revendication 1, **caractérisé en ce que** :

- les x polymères présentent des taux de réticulation différents, au moins l'un des x polymères présentant un taux de réticulation x1 et au moins un des x polymères présentant un taux de réticulation x2, x1 étant supérieur ou égal à x2 ; ou
- les x polymères présentent des taux de réticulation identiques et des masses moléculaires différentes.

**3.** Hydrogel selon l'une des revendications 1 à 2, **caractérisé en ce que** les polysaccharides sont choisis dans le groupe constitué par l'acide hyaluronique, le kératane, l'héparine, la cellulose et les dérivés de cellulose, l'acide alginique, le xanthane, la carraghénane, le chitosane et la chondroitine et leurs sels biologiquement acceptables.

**4.** Hydrogel selon l'une quelconque des revendications 1 à 3, **caractérisé en ce que** :

- les x polysaccharides sont choisis dans le groupe constitué par l'acide hyaluronique et ses sels biologiquement acceptables ; ou
- au moins un des x polysaccharides est choisi dans le groupe constitué par les dérivés de cellulose et leurs sels biologiquement acceptables ; ou
- au moins un des x polysaccharides est choisi dans le groupe constitué par la chondroitine et ses sels biologiquement acceptables ; ou
- au moins un des x polysaccharides est choisi dans le groupe constitué par le chitosane et ses sels biologiquement acceptables.

**5.** Hydrogel selon l'une quelconque des revendications précédentes, **caractérisé en ce que** x est égal à 2.

**6.** Hydrogel selon la revendication 5, **caractérisé en ce que** :

- le premier des x polymères est l'acide hyaluronique et le deuxième est choisi dans le groupe constitué par la chondroïtine sulfate et ses sels, le chitosane et ses sels et dérivés, les dérivés de cellulose et leurs sels et les acides alginiques ; ou
- le premier des x polysaccharides est choisi dans le groupe constitué par l'acide hyaluronique et ses sels, les dérivés de cellulose et leurs sels et la xanthane et le deuxième est choisi dans le groupe constitué par la chondroïtine sulfate et ses sels, le chitosane et ses sels et dérivés, les dérivés de cellulose et leurs sels et les acides alginiques.

**7.** Hydrogel selon l'une quelconque des revendications précédentes, **caractérisé en ce qu'**il peut comprendre un ou plusieurs principe(s) actif(s) choisi(s) parmi les anti-oxydants, les antiseptiques, les anti-inflammatoires et les anesthésiques locaux seuls ou en combinaison.

**8.** Hydrogel selon la revendication 7, **caractérisé en ce que** :

- les anti-oxydants sont choisis parmi le mannitol et le sorbitol, seul ou en combinaison ; ou
- l'anesthésiant local est de la lidocaïne.

**9.** Procédé de préparation d'un hydrogel cohésif monophasique biodégradable selon l'une des revendications précédentes, **caractérisé en ce qu'**il comprend au moins les étapes de :

- réticulation d'un premier polymère indépendamment de l'autre à un taux de réticulation x1,
- réticulation d'un deuxième polymère indépendamment de l'autre à un taux de réticulation x2,
- interpénétration par mélange intime des deux polymères,
- hydratation,
- interpénétration finale par mélange final après hydratation.

**10.** Procédé selon la revendication 9, **caractérisé en ce qu'**il comprend en outre x étapes de réticulation des x polymères indépendamment les uns des autres avant le mélange des x polymères réticulés.

**11.** Procédé selon l'un des revendications 9 à 10, **caractérisé en ce que** les réticulations sont effectuées par action d'un agent réticulant polyfonctionnel choisi dans le groupe des époxy bi ou poly fonctionnels, de la divinyl sulfone, des carbodiimides ou du formadéhylde.

**12.** Procédé selon l'une des revendications 9 à 11, **caractérisé en ce que** les agents réticulants mis en oeuvre dans les étapes de réticulation sont identiques ou différents.

**13.** Procédé selon l'une des revendications 9 à 12, **caractérisé en ce que** le taux de réticulation x1 est supérieur ou égal au taux de réticulation x2.

**14.** Procédé selon l'une des revendications 9 à 13, **caractérisé en ce que** les taux de réticulation sont compris entre 0,02 et 0,4, de préférence entre 0,08 et 0,2.

**15.** Utilisation d'un hydrogel selon l'une quelconque des revendications 1 à 8, pour la formulation d'une composition de viscosupplémentation ou pour la formulation d'une composition pour le comblement des rides.

**16.** Kit comprenant un hydrogel selon l'une quelconque des revendications 1 à 8, conditionné en seringue stérile.

**Patentansprüche**

**1.** Kohäsives monophasisches biologisch abbaubares Hydrogel, **dadurch gekennzeichnet, dass**

- es gebildet ist aus einer homogenen Mischung aus x Polymeren, die ausgewählt sind aus identischen oder unterschiedlichen Polysaccharaiden, die unabhängig voneinander in Form eines monophasischen Hydrogels vernetzt sind, vor ihrer gegenseitigen Durchdringung durch innige Vermischung, die zwischen den Ketten schwache Bindungen erzeugt, wobei die vernetzten Polymere in Wasser unlöslich sind aber miteinander mischbar sind, und
- x von 2 bis 5 reicht,
- die x vernetzten Polymere voneinander nicht trennbar sind,
- die x Polymere unterschiedliche Vernetzungsgrade aufweisen, oder
- die x Polymere identische Vernetzungsgrade und verschiedene Molekulargewichte aufweisen.

**2.** Hydrogel nach Anspruch 1, **dadurch gekennzeichnet, dass**

- die x Polymere unterschiedliche Vernetzungsgrade aufweisen, mindestens eines der x Polymere einen Vernetzungsgrad x1 aufweist und wenigstens eines der x Polymere einen Vernetzungsgrad x2 aufweist, wobei x1 größer oder gleich x2 ist; oder
- die x Polymere identische Vernetzungsgrade und unterschiedliche Molekulargewichte aufweisen.

**3.** Hydrogel nach einem der Ansprüche 1 bis 2, **dadurch gekennzeichnet, dass** die Polysaccharide ausgewählt sind aus der Gruppe bestehend aus Hyaluronsäure, Keratan, Heparin, Zellulose und Zellulosederivate, Alginsäure, Xanthan, Karragenan, Chitosan und Chondroitin und den biologisch akzeptablen Salzen davon.

**4.** Hydrogel nach einem der Ansprüche 1 bis 3, **dadurch gekennzeichnet, dass**

- die x Polysaccharide ausgewählt sind aus der Gruppe bestehend aus Hyaluronsäure und den biologisch akzeptablen Salze davon; oder
- wenigstens eines der x Polysaccharide ausgewählt ist aus der Gruppe bestehend aus Zellulosederivaten und den biologisch akzeptablen Salze davon; oder
- wenigstens eines der x Polysaccharide ausgewählt ist aus der Gruppe bestehend aus Chondroitin und den biologisch akzeptablen Salze davon; oder
- wenigstens eines der x Polymere ausgewählt ist aus der Gruppe bestehend aus Chitosan und den biologisch akzeptablen Salzen davon.

**5.** Hydrogel nach einem der vorangehenden Ansprüche, **dadurch gekennzeichnet, dass** x gleich 2 ist.

**6.** Hydrogel nach Anspruch 5, **dadurch gekennzeichnet, dass**

- das erste der x Polymeren Hyaluronsäure ist und das zweite ausgewählt ist aus der Gruppe bestehend aus Chondroitinsulfat und Salzen davon, Chitosan und Salzen und Derivaten davon, Zellulosederivaten und Salzen davon und Alginsäuren; oder

- das erste der x Polysaccharide ausgewählt ist aus der Gruppe bestehend aus Hyaluronsäure und Salzen davon, Zellulosederivaten und Salzen davon und Xanthan, und das zweite ausgewählt ist aus der Gruppe bestehend aus Chondroitinsulfat und Salzen davon, Chitosan und Salzen und Derivaten davon, Zellulosederivaten und deren Salzen und Alginsäuren.

7. Hydrogel nach einem der vorangehenden Ansprüche, **dadurch gekennzeichnet, dass** es ein oder mehrere aktive(s) Prinzip/Prinzipien umfassen kann, das/die ausgewählt ist/sind aus Antioxidanzien, Antiseptika, Antiphlogistika und Lokalanästhetika, alleine oder in Kombination.

8. Hydrogel nach Anspruch 7, **dadurch gekennzeichnet, dass**

   - die Antioxidantien ausgewählt sind aus Mannitol und Sorbitol, alleine oder in Kombination; oder
   - das Lokalanästhetikum Lidocain ist.

9. Verfahren zur Herstellung eines kohäsiven monophasischen biologisch abbaubaren Hydrogels nach einem der vorangehenden Ansprüche, **dadurch gekennzeichnet, dass** es die folgenden Schritte umfasst:

   - Vernetzen eines ersten Polymers unabhängig von dem anderen zu einem Vernetzungsgrad x1,
   - Vernetzen eines zweiten Polymers unabhängig von dem anderen zu einem Vernetzungsgrad x2,
   - Durchdringen der beiden Polymere durch inniges Vermischen,
   - Hydratisieren,
   - Enddurchdringen durch abschließendes Vermischen nach Hydratisieren.

10. Verfahren nach Anspruch 9, **dadurch gekennzeichnet, dass** es unter anderem x Vernetzungsschritte der x Polymere umfasst, die unabhängig voneinander sind, vor dem Vermischen der x vernetzten Polymeren.

11. Verfahren nach einem der Ansprüche 9 bis 10, **dadurch gekennzeichnet, dass** die Vernetzungen bewirkt werden durch ein polyfunktionelles Vernetzungsmittel, das ausgewählt ist aus der Gruppe der bi- oder polyfunktionellen Epoxy, Divinylsulfon, Carbodiimide oder Formaldehyd.

12. Verfahren nach einem der Ansprüche 9 bis 11, **dadurch gekennzeichnet, dass** die in den Vernetzungsschritten umgesetzten Vernetzungsmittel identisch oder verschieden sind.

13. Verfahren nach einem der Ansprüche 9 bis 12, **dadurch gekennzeichnet, dass** der Vernetzungsgrad x1 größer oder gleich ist dem Vernetzungsgrad x2.

14. Verfahren nach einem der Ansprüche 9 bis 13, **dadurch gekennzeichnet, dass** die Vernetzungsgrade von 0,02 bis 0,4, bevorzugterweise von 0,08 bis 0,2 reichen.

15. Verwendung eines Hydrogels nach einem der vorangehenden Ansprüche 1 bis 8, für die Formulierung einer Viskosupplementationszusammensetzung oder für die Formulierung einer Zusammensetzung für das Ausfüllen von Falten.

16. Kit umfassend ein Hydrogel nach einem der Ansprüche 1 bis 8, verpackt in eine sterile Spritze.

**Claims**

1. Biodegradable single-phase cohesive hydrogel, wherein:

   - it is composed of a homogeneous mixture of x polymers chosen from identical or different polysaccharides, crosslinked independently of one another in a single-phase hydrogel form, prior to their interpenetration by intimate mixing generating weak interchain bonds between the polymers, said crosslinked polymers being insoluble in water and miscible with one another, and
   - x being between 2 and 5,
   - the x crosslinked polymers being indissociable from one another,
   - the x polymers having different degrees of crosslinking, or
   - the x polymers having identical degrees of crosslinking and different molecular weights.

2. Hydrogel according to Claim 1, wherein:

   - the x polymers exhibit different degrees of crosslinking, at least one of the x polymers exhibiting a degree of crosslinking x1 and at least one of the x polymers exhibiting a degree of crosslinking x2, x1 being greater than or equal to x2; or
   - the x polymers exhibit identical degrees of crosslinking and different molecular weights.

3. Hydrogel according to either of Claims 1 and 2, wherein the polysaccharides are selected from the group consisting of hyaluronic acid, keratan, heparin, cellulose and cellulose derivatives, alginic acid, xanthan, carrageenan, chitosan and chondroitin and their biologically acceptable salts.

4. Hydrogel according to any one of Claims 1 to 3, wherein:

   - the x polysaccharides are selected from the group consisting of hyaluronic acid and its biologically acceptable salts; or
   - at least one of the x polysaccharides is selected from the group consisting of cellulose derivatives and their biologically acceptable salts; or
   - at least one of the x polysaccharides is selected from the group consisting of chondroitin and its biologically acceptable salts; or
   - at least one of the x polysaccharides is selected from the group consisting of chitosan and its biologically acceptable salts.

5. Hydrogel according to any one of the preceding claims, wherein x is equal to 2.

6. Hydrogel according to Claim 5, wherein:

   - the first of the x polymers is hyaluronic acid and the second is selected from the group consisting of chondroitin sulfate and its salts, chitosan and its salts and derivatives, cellulose derivatives and their salts and alginic acids; or
   - the first of the x polysaccharides is selected from the group consisting of hyaluronic acid and its salts, cellulose derivatives and their salts and xanthan and the second is selected from the group consisting of chondroitin sulfate and its salts, chitosan and its salts and derivatives, cellulose derivatives and their salts and alginic acids.

7. Hydrogel according to any one of the preceding claims, wherein it can comprise one or more active principle(s) selected from antioxidants, antiseptics, antiinflammatories and local anesthetics, alone or in combination.

8. Hydrogel according to Claim 7, wherein:

   - the antioxidants are selected from mannitol and sorbitol, alone or in combination; or
   - the local anesthetic is lidocaine.

9. Process for the preparation of a biodegradable single-phase cohesive hydrogel according to one of the preceding claims, wherein it comprises at least the stages of:

   - crosslinking a first polymer independently of the other to a degree of crosslinking x1,
   - crosslinking a second polymer independently of the other to a degree of crosslinking x2,
   - interpenetration by intimate mixing of the two polymers,
   - hydration,
   - final interpenetration by final mixing after hydration.

10. Process according to Claim 9, wherein it additionally comprises x stages of crosslinking the x polymers independently of one another before mixing the x crosslinked polymers.

11. Process according to either of Claims 9 and 10, wherein the crosslinkings are carried out by the action of a polyfunctional crosslinking agent selected from the group consisting of bi- or polyfunctional epoxy compounds, divinyl sulfone, carbodiimides and formaldehyde.

12. Process according to one of Claims 9 to 11, wherein the crosslinking agents employed in the crosslinking stages are identical or different.

13. Process according to one of Claims 9 to 12, wherein the degree of crosslinking x1 is greater than or equal to the degree of crosslinking x2.

14. Process according to one of Claims 9 to 13, wherein the degrees of crosslinking are between 0.02 and 0.4, preferably between 0.08 and 0.2.

15. Use of a hydrogel according to any one of Claims 1 to 8, in the formulation of a viscosupplementation composition or in the formulation of a composition for filling in wrinkles.

16. Kit comprising a hydrogel according to any one of Claims 1 to 8, packaged in a sterile syringe.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2865737
- FR 2861734
- WO 2005061611 A
- US 6224893 B
- FR 2733427
- US 4582865 A
- US 4716154 A
- GB 2151244 A
- WO 200046253 A
- EP 0466300 A